# EUROPEAN PATENT APPLICATION

(11) **EP 4 235 183 A2**
(43) Date of publication of application: **30.08.2023**
(21) Application number: 23158500.1
(22) Date of filing: 24.02.2023
(51) Int. Cl.: G01N 35/00, G01N 35/04, G01N 35/02

(54) **SPECIMEN PREPARATION APPARATUS, SPECIMEN PROCESSING APPARATUS, SPECIMEN PREPARATION METHOD ANDSPECIMEN PROCESSING METHOD**

(30) Priority: 28.02.2022 JP 2022030373; 28.02.2022 JP 2022030375
(71) Applicant: SYSMEX CORPORATION, Kobe-shi Hyogo 651-0073 (JP)
(72) Inventor: Sasaki, Yuto, Kobe-shi, Hyogo (JP); Shohmi, Keiichiro, Kobe-shi, Hyogo, 651-0073 (JP); Iguchi, Kazuyuki, Kobe-shi, Hyogo, 651-0073 (JP)
(74) Representative: Hoffmann Eitle

(57) **Abstract**

A specimen preparation apparatus 100 includes a holder 21 that holds a sample container 13 and a centrifuge tube 12 provided by a user, a centrifugation section 30 that centrifuges a specimen stored in the centrifuge tube 12, a transfer unit 40 that transfers the centrifuge tube 12 between the holder 21 and the centrifugation section 30, and a dispenser 50 that dispenses a sample stored in the sample container 13 or a specimen prepared based on a sample into the centrifuge tube 12 held in the centrifugation section 30. The transfer unit 40 transfers the centrifuge tube 12 storing a centrifuged sample or specimen to the holder 21.

## Description

### TECHNICAL FIELD

This invention relates to a specimen preparation apparatus, a specimen processing apparatus, a specimen preparation method and a specimen processing method.

### BACKGROUND ART

Patent Document 1 discloses a specimen preparation apparatus provided with a centrifuge. In the specimen preparation apparatus of Patent Document 1, a user performs an operation to set a centrifuge tube in a centrifuge.

[Patent document 1] Japanese Patent Publication No. 2021-162321

### SUMMARY

In the above Patent Document 1, there is room for improvement from a viewpoint of automation because a user needs to perform an operation to set a centrifuge tube in a centrifuge.

One object of this invention is to achieve further automation of a specimen preparation apparatus provided with a centrifuge.

In order to achieve the above object, a specimen preparation apparatus (100) according to a first invention is a specimen preparation apparatus (100) to prepare a measurement specimen from a specimen and includes a holder (20) that holds a centrifuge tube (12), a transfer unit (40) that transfers the centrifuge tube (12) held by the holder (20), a dispenser (60) that dispenses a specimen into the centrifuge tube (12) before or after transfer by the transfer unit (40), and a centrifugation section (30) that holds the centrifuge tube (12) transferred by the transfer unit (40) and centrifuges the specimen dispensed into the centrifuge tube by rotation.

The specimen preparation apparatus (100) according to a first invention, as described above, includes the transfer unit (40) that transfer the centrifuge tube (12) between the holder (20) and the centrifugation section (30). Since the transfer unit (40) allows the centrifuge tube (12) to be transferred to the centrifugation section (30), an operation that a user sets the centrifuge tube (12) in the centrifugation section (30) is reduced, and further automation is achieved.

A specimen processing apparatus (100) according to a second invention includes a container transport mechanism (22) that includes a holder (20) to hold a plurality of containers capable of storing specimens and a drive unit (21a) to move the holder (20), a processing section that processes containers (10a to 10c) or a specimen in the containers (10a to 10c), and a controller (90), wherein the controller (90) controls the container transport mechanism (22) and the processing section, so that the plurality of containers (10a to 10c) are moved in the same direction by the drive unit (21a), at least one type of container (10a to 10c) is provided at a predetermined position, and at least one type of container (10a to 10c) or a specimen in at least one type of container (10a to 10c) is performed with a predetermined process.

The specimen processing apparatus according to the second invention can simplify the container transport mechanism and reduce the size of the apparatus since multiple types of containers are not necessary to be transported individually.

A method of preparing a specimen according to a third invention includes transferring a centrifuge tube (12) provided by a user is transferred to a centrifugation section (30), dispensing a sample into the centrifuge tube (12) transferred to the centrifugation section (30), and centrifuging the sample stored in the centrifuge tube (12) by the centrifugation section (30).

According to the method of preparing a specimen according to the third invention, an operation that the user sets the centrifuge tube (12) to the centrifugation section (30) can be reduced, and further automation can be achieved.

A method of processing a specimen according to a fourth invention is a method of processing a specimen using the specimen processing apparatus (100) that includes: the container transport mechanism (22), which includes the holder (20) to hold a plurality of containers (10) capable of storing a specimen and the drive unit (21a) to move the holder (20); the processing section that processes the containers (10a to 10c) or a specimen in the containers (10a to 10c); and the controller (90), wherein a plurality of containers (10a to 10c) are moved in the same direction by the drive unit (21a), at least one type of container is provided at a predetermined processing position, and at least one type of container (10a to 10c) or a specimen in at least one type of container (10a to 10c) is performed with a predetermined process.

According to the method of processing a specimen according to the fourth invention, since multiple types of containers do not need to be transported individually, the container transport mechanism can be simplified, and the apparatus can be downsized.

According to the present invention, further automation of specimen preparation involving centrifugation can be achieved. Also, according to the present invention, a container transport mechanism can be simplified, and an apparatus can be downsized.

### BRIEF DESCRIPTION OF DRAWINGS

FIG.1 is a schematic diagram illustrating an overview of a first configuration example of a specimen preparation apparatus.
FIG. 2 is a perspective view illustrating a transfer mechanism of the specimen preparation apparatus.
FIG.3 is a cross-sectional view along a III-III line of FIG. 2.
FIG. 4 is a diagram illustrating a case of a centrifuge tube.
FIG. 5 is a schematic diagram illustrating a transfer unit of the specimen preparation apparatus.
FIG. 6 is a first plan view illustrating a lid of a centrifugation section of the specimen preparation apparatus.
FIG. 7 is a second plan view illustrating the lid of the centrifugation section of the specimen preparation apparatus.
FIG. 8 is a third plan view illustrating the lid of the centrifugation section of the specimen preparation apparatus.
FIG. 9 is a diagram illustrating a specific example of a specimen preparation process.
FIGS. 10A, 10B, and 10C are diagrams illustrating a movement of a rack when transferring a centrifuge tube by the specimen preparation apparatus.
FIGS. 11A, 11B, 11C, and 11D are diagrams illustrating a movement of a rack when dispensing a sample by the specimen preparation apparatus.
FIGS. 12A, 12B, 12C, 12D, and 12E are diagrams illustrating a movement of a rack when a processing section performs a process in the specimen preparation apparatus.
FIGS. 13A, 13B, 13C, and 13D are diagrams illustrating a movement of a rack after the processing section performs a process in the specimen preparation apparatus.
FIG. 14 is a flow diagram to explain an operation process of the specimen preparation apparatus.
FIG. 15 is a schematic diagram illustrating an overview of another configuration example of the specimen preparation apparatus.
FIGS. 16A, and 16B is a diagram illustrating a rotary table when a sample is dispensed by the specimen preparation apparatus and processed by the processing section.
FIGS. 17A and17B are diagrams illustrating a rotary table when a centrifugation section performs a process in the specimen preparation apparatus.

### DETAILED DESCRIPTION

Embodiments are described on the basis of a drawing.

### [An overview of a specimen preparation apparatus]

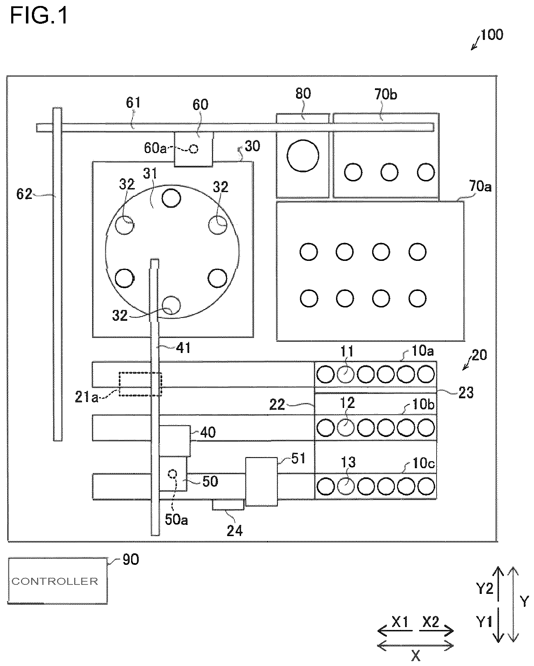

First, with reference to FIG. 1, an overview of a specimen preparation apparatus 100 as a specimen processing apparatus according to an embodiment is described.

The specimen preparation apparatus 100 is an apparatus to prepare a measurement specimen for measurement by a flow cytometer by performing a pretreatment on a blood sample. The measurement specimen is an immunostained specimen of a white blood cell derived from a blood sample. More specifically, the measurement specimen is prepared in which a cell surface antigen (e.g., CD4, CD25, CD62L) and an intracellular antigen (e.g., FOXP3) of a T cell included in the blood sample are labeled by a labeled antibody.

FIG. 1 is a plan view illustrating an overview of a configuration of the specimen preparation apparatus 100. As illustrated in FIG. 1, the specimen preparation apparatus 100 includes a rack transporter (holder) 20 provided on a front side (Y1 direction side) of the apparatus. The rack transporter 20 includes a rack 10a that holds a plurality of processing containers 11, a rack 10b that holds a plurality of centrifuge tubes 12, and a rack 10c that holds a plurality of sample containers 13 in which blood samples are stored. The rack transporter 20 includes a container transport mechanism 22. The racks 10a to 10c are spaced along a Y axis so that rows of containers held by each rack are aligned along an X axis, i.e., a longitudinal direction of the rack is along the X axis. In an embodiment of FIG. 1, each rack is fixed in such a way that it cannot be removed from the rack transporter 20. A user sets the containers 11 to 13 on each rack fixed to the rack transporter 20. A processing container 11 is used for separating a specific component in a specimen.

The rack transporter 20 includes a common drive unit 21a to move the racks 10a to 10c integrally along an X direction.

The rack transporter 20 includes a magnet 23 that is provided between the rack 10a to hold the processing container 11 and the rack 10b to hold the centrifuge tube (reaction container) 12. The magnet 23 is used to collect a complex including red blood cells and a magnetic particle (solid phase substance) formed in the processing container 11 as described below.

The specimen preparation apparatus 100 includes a centrifugation section 30. The centrifugation section 30 includes a rotor 31 that rotates at a high speed and a plurality of holders 32 that are provided around the periphery of the rotor 31. The holder 32 has, for example, a cylindrical shape and can receive and hold the centrifuge tube 12 therein. Also, the holder 32 holds the centrifuge tube 12 with its opening facing upward when the rotor 31 is stopped.

The specimen preparation apparatus 100 includes a transfer unit 40 that transfers the centrifuge tube 12 between the rack transporter 20 and the centrifugation section 30. The transfer unit 40 is movably supported in a Y direction by a transfer axis 41. Also, the transfer unit 40 is movable in a Z direction (vertical direction).

The specimen preparation apparatus 100 includes a dispenser 50. The dispenser 50 is movably supported in the Y direction on the transfer axis 41 common to the transfer unit 40. Also, the dispenser 50 is movable in the Z direction (vertical direction). The dispenser 50 includes a pipette 50a, dispenses a sample stored in the sample container 13 into the processing container 11 using the pipette 50a.

The specimen preparation apparatus 100 includes an agitator 51 that agitates a blood sample. The agitator 51 agitates a blood sample in the container 13 by holding and removing the sample container 13 from the rack 10c and inverting and agitating the blood sample.

The specimen preparation apparatus 100 includes a dispenser 60. The dispenser 60 is movably supported in the X direction by a transfer axis 61. Also, the transfer axis 61 is movably supported in the Y direction by a transfer axis 62. As a result, the dispenser 60 is movable in a horizontal direction in the apparatus. Also, the dispenser 60 is movable in the Z direction (vertical direction). The dispenser 60 includes a pipette 60a, and dispenses a reagent installed in a reagent setting part 70a and a reagent setting part 70b to the processing container 11 of the rack transporter 20 or the centrifuge tube 12 in the centrifugation section 30. The dispenser 60 further aspirates a supernatant in the processing container 11, in a state in which magnetic particles are attracted by the magnet 23, and dispenses the supernatant into the centrifuge tube 12 transferred to the centrifugation section 30. The dispenser 60 discharges the reagent into the processing container 11, which is moved to a reagent discharging position by the drive unit 21a.

The specimen preparation apparatus 100 includes reagent setting parts 70a and 70b. The reagent setting part 70a includes a cold storage and holds the reagent at a low temperature. The reagent setting part 70b holds the reagent at a normal temperature.

The specimen preparation apparatus 100 includes a nozzle cleaner 80. The nozzle cleaner 80 cleans a nozzle of the dispenser 60.

The specimen preparation apparatus 100 includes a controller 90 that controls each part of the apparatus. The controller 90 includes a processor and a memory. The processor is configured with, for example, a CPU. The memory may include a memory and a storage. The processor controls each part of the specimen preparation apparatus 100 by executing a program stored in the memory.

The reagent setting part 70a includes a lid and a shutter member. The reagent setting part 70a is a box-shaped member with a lid comprising a top surface that can be opened and closed. The lid is provided to cover an upper part of the reagent setting part 70a. In the lid, a plurality of insertion holes through which nozzles are passed is formed at a position above the plurality of reagent containers. In the shutter member, an insertion hole of the nozzle and a shielding section are formed. The shutter member is provided on the lid so as to be opened and closed integrally with the lid.

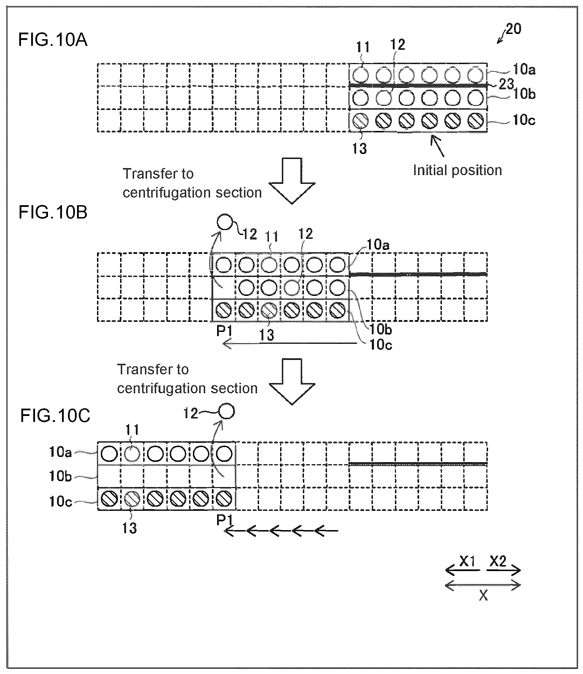

In a layout of the specimen preparation apparatus 100 illustrated in FIG. 1, parts to be accessed by a user for operation of the apparatus, specifically, the reagent setting parts 70a, 70b and an initial positions (see FIG. 10) where containers are set in the racks 10a to 10c held by the tack transporter 20 are provided concentrating on a right side (X1 direction side) of the apparatus. In particular, an initial position of the rack transporter 20, which is frequently accessed by the user, is in front (Y1 direction side) of the right side (X1 direction side), while the centrifugation section 30 is provided at the back (Y2 direction side) of a left side (X2 direction side). In other words, the centrifugation section 30 and the rack transporter 20 are separated by a predetermined distance, and the layout is designed to reduce the frequency of a user putting his/her hand near the centrifugation section 30. Since the rotor 31 of the centrifugation section 30 is structured to rotate at a high speed, it is preferable to keep the user's hands as far away from the rotor 31 as possible in order to improve safety. The specimen preparation apparatus 100 does not require the user to access the centrifugation section 30 by allowing the centrifuge tube 12 to be set into the centrifugation section 30 by the transfer unit 40. Furthermore, the rack transporter 20, which is accessed by the user to set the centrifuge tube 12 into the apparatus, is provided away from the centrifugation section 30, further reducing the possibility of the user accidentally placing his or her hand near the operating centrifugation section 30.

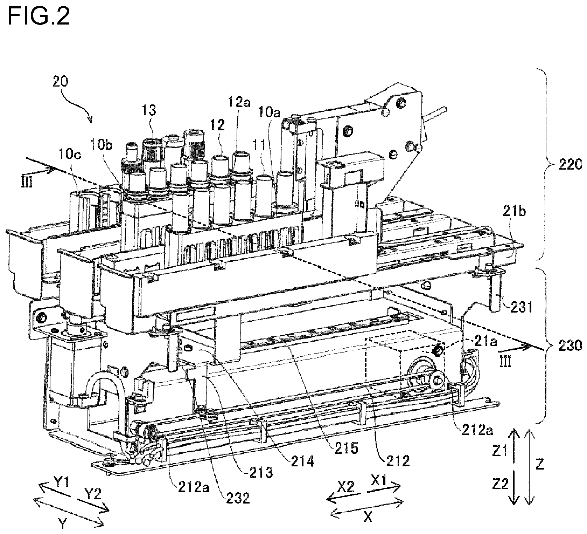

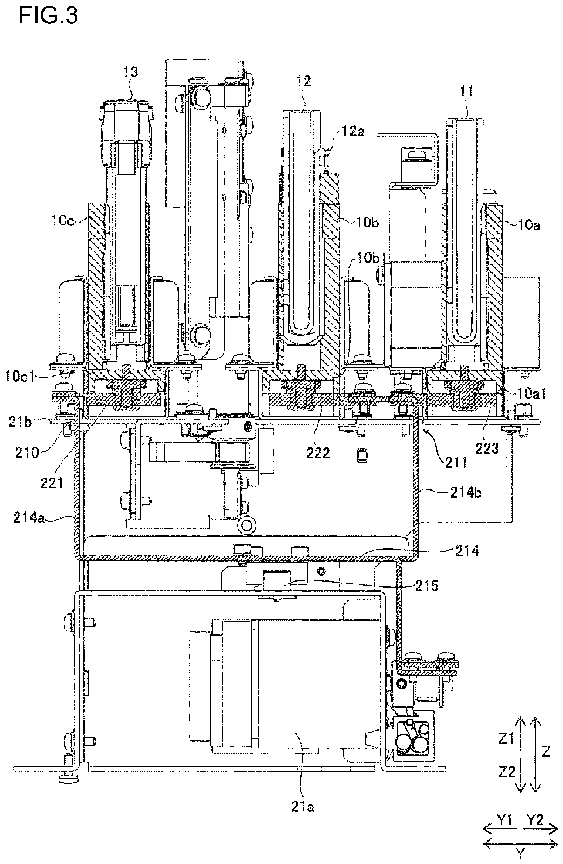

Referring to FIG. 2 and FIG. 3, the rack transporter 20 is described. FIG. 2 is a perspective diagram illustrating the rack transporter 20 viewed from the rear (Y2 direction side). The rack transporter 20 includes an upper portion that includes a base 21b, on which the racks 10a to 10c are installed, and a lower portion 230 that is provided below the upper portion 220 and generates and transmits a driving force to move the racks 10a to 10c. The upper portion 220 and the lower portion 230 are provided with a space between a sheet metal 231 on the left side (X1 direction side) and a sheet metal 232 on the right side (X2 direction).

The lower portion 230 of the rack transporter 20 includes a drive unit 21a comprising a motor, a pair of pulleys 212a, and a belt 212. An output axis of the drive unit 21a is connected to the pulley 212a on the left side (X1 direction side). The belt 212 extends along the X axis, and both ends are crossed over the pair of pulleys 212a. A connecting member 213 is fixed to the belt212. The connecting member 213 is connected to a slider 214. The slider 214 is movable in the X direction along a rail 215.

FIG. 3 is an arrowhead cross section of the rack transporter 20 in FIG. 2 along a III-III line. The slider 214 includes a side wall 214a that is provided on a Y1 direction side and extends upwardly and a side wall 214b that is provided on a Y2 direction side and also extends upwardly. The base 21b is provided with slits 210 and 211 extending along the X direction. The side wall 214a projects upwardly from the bottom of the base 21b via the slit 210. The side wall 214b projects upwardly from the bottom of the base 21b via the slit 211.

An upper end part of the side wall 214a projecting out of the slit 210 is connected to a fastener 221 that is screwed to a bottom part 10c1 of the rack 10c. The upper end part of the side wall 214b projecting from the slit 211 is connected to the fastener 221 that is screwed to the bottom part 10c1 of the rack 10c, a fastener 222 that is screwed to a bottom part 10b1 of the rack 10b, and a fastener 223 that is screwed to a bottom part 10a1 of the rack 10a.

In this configuration, when side walls 214a, 214b of the slider 214 move in the X axis direction via the slits 210, 211 by driving of the drive unit 21a described above, the racks 10a to 10c connected to the side walls 214a, 214b move integrally in the X axis direction. In this embodiment, the slider 214 of the rack transporter 20 and the racks 10a to 10c are screwed and secured by the fasteners 221, 222, and 223, but screw fixing is not required as long as the slider 214 and the racks 10a to 10c are interlocked by engaging with each other. For example, projections convexly provided downwardly on the bottom of the racks 10a to 10c and holes provided in the upper end parts of the side walls 214a, 214b may be engaged with each other.

As illustrated in FIG. 2 and FIG. 3, the centrifuge tube 12 is set in the rack 10b while stored in a case 12a. The transfer section 40, which is described later, is configured to remove the centrifuge tube 12 with the case 12a from the rack 10b and transfers the centrifuge tube 12 with the case 12a to the centrifugation section 30.

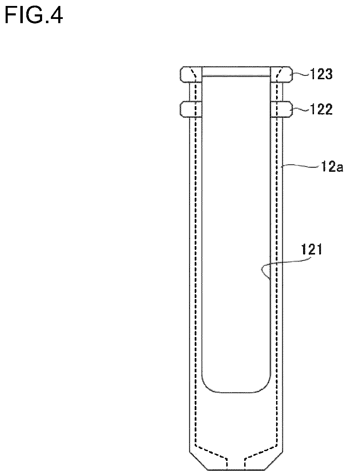

FIG. 4 is a front view illustrating the case 12a. The case 12a has a notch 121 and flanges 122 and 123. The case 12a is formed of a thermally conductive metallic material, e.g., aluminum material. By forming the case 12a from a material having high thermal conductivity, the adjusted temperature in the centrifugation section 30 is transferred to the specimen in the centrifuge tube 12, thereby facilitating temperature control of the specimen. The notch 121 allows identification information, such as a bar code or a two-dimensional code imprinted on a side of the centrifuge tube 12, to be read by a reader, which is not illustrated in the figure, while the centrifuge tube 12 is stored in the case 12a. The flanges 122, 123 function as hooks that prevent the case 12a from falling out when the transfer unit 40 grips and transfers the case 12a.

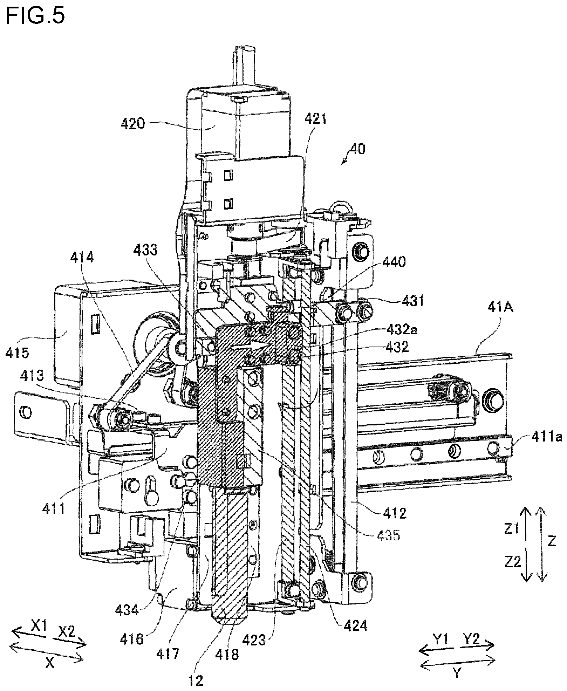

Referring to FIG. 5, a configuration of the transfer unit 40 is described. FIG. 5 is a perspective view illustrating a configuration of the transfer unit 40. In FIG. 5, in order to clarify explanation, a part that moves integrally is illustrated with the same hatching. Also, the centrifuge tube 12 is hatched to make a position of the centrifuge tube 12 easier to understand.

The transfer unit 40 is attached to a sheet metal 41A constituting a transfer axis 41 in a Y axis and Z axis movable manner. A Y axis motor 415 is attached to the sheet metal 41A. A belt 414 is stretched over an output axis of the Y axis motor 415. Also, a rail 411a extending horizontally is attached to the sheet metal 41A. A sheet metal 411 is slidably attached to the rail 411a. A part of the sheet metal 411 is fastened to the belt 414 by a fastener 413. Therefore, the belt is driven by the Y axis motor 415, and the sheet metal 411 moves along the Y axis accordingly.

A sheet metal 417 to support a part involved in a Z axis movement described below is attached to the sheet metal 411. The sheet metal 417 is a vertical plate extending in a vertical direction, and a Z axis motor 416 is attached on a lower back (X1 direction side) of the sheet metal 417. An open/close motor 420 is provided above the sheet metal 417. Also, a longitudinal rail 418 is attached to a front side of the sheet metal 417 (X2 direction side). An output of the Z axis motor 416 is transmitted to a belt 412 that is vertically bridged. A sheet metal 431 is fixed to the belt 412. The sheet metal 431 is slidably attached to a rail 418. The sheet metal 431 has a rail 433 extending horizontally. A slide section 432 is slidably attached to the rail 433. The slide section 432 is biased toward the Y2 direction along the rail 433 by a spring member 440 that is horizontally provided between the sheet metal 431 and the slide section 432, as illustrated by a white arrow in FIG. 5.

At a lower end of the slide section 432 on the Y1 direction side, a first gripper 434 that consists of one of a pair of grippers to grip the upper part of the centrifuge tube 12 is attached. In FIG. 5, as the slide section 432 and the first gripper 434 are illustrated with the same hatchings, these parts move in unison. In other words, when the slide section 432 moves in the Y1 direction as described below, the first gripper 434 also moves in the Y1 direction accordingly. A second gripper 435 that consists of the other gripper is fixed to the sheet metal 431 described above. In other words, with respect to the second gripper 435 fixed to the sheet metal 431, the first gripper is movable in the Y1 direction.

At a rear (Y2 direction side) end of the slide section 432, a thick contacting section 432a is provided on the X2 direction side. The open/close motor 420 provided above the sheet metal 417 has its output axis connected to the belt 421. When the driving force of the open/close motor 420 is transmitted to the belt 421, a first rod 423, which is provided to extend vertically against the sheet metal 417 and to be rotatable around an axis, rotates. The upper and lower ends of the first rod 423 are connected to a second rod 424, which is parallel to the first rod 423 and extends in the vertical direction, and when the first rod 423 rotates about its own axis, the second rod 424 rotates about the first rod 423, as illustrated by an arrow in FIG. 5. A part of the sheet metal 431 described earlier, that is, a part between an end of the Y2 direction side fixed to the belt 412 and a part slidably fixed to the rail 418 extends between the first rod 423 and the second rod 424.

When the second rod 424 rotates about the first rod 423, the contacting section 432a of the sliding section 432 is pushed in the Y1 direction by the second rod 424. As a result, the slide section 432 moves in the Y1 direction against the force of the spring member 440. As described earlier, the slide section 432 and the first gripper 434 move integrally, and by the first gripper 434 moving in the Y1 direction, a space between the first gripper 434 and the second gripper 435 opens, and the centrifuge tube 12 can be gripped in the meantime.

The Z axis motor 416 is connected to a pair of grippers 434, 435 via the belt 412, sheet metal 431, and slide section 432. When the Z axis motor 416 is driven, the sheet metal 431 fixed to the belt 412 moves in the vertical direction. As the sheet metal 431 moves in the vertical direction, the grippers 434, 43 5 move in the vertical direction along the Z axis.

When moving the centrifuge tube 12 from the rack transporter 20 to the centrifugation section 30, the Z axis motor 416 and the open/close motor 420 lower the grippers 434, 435 to grip the centrifuge tube 12 in the rack transporter 20 and move the griped centrifuge tube 12 upward. The grippers 434, 435, which grip the centrifuge tube 12, are moved to an upper side of the centrifugation section 30 by the drive of the Y axis motor 415. After moving the grippers 434 and 435 to the upper side of the centrifugation section 30, the Z axis motor 416 lowers the centrifuge tube 12, and the centrifuge tube 12 is inserted into the holder 32 of the centrifugation section 30. The open/close motor 420 opens the grippers 434, 435, and the gripping is released. When the centrifuge tube 12 that stores the specimen prepared in the centrifugation section 30 is returned to the rack transporter 20, the reverse operation is performed. That is, by the Z axis motor 416 and the open/close motor 420, the grippers 434, 435 are lowered to grip the centrifuge tube 12 in the centrifugation section 30, and the gripped centrifuge tube 12 is moved upward. The grippers 434, 435, that grip the centrifuge tube 12, are moved to the upper side of the rack transporter 20 by the drive of the Y axis motor 415. After moving the grippers 434 and 435 to the upper side of the rack transporter 20, by the Z axis motor 416, the centrifuge tube 12 is lowered, and the centrifuge tube 12 is inserted into an original holding position of the rack 10b in the rack transporter 20. The grippers 434, 435 are opened by the open/close motor 420, and the gripping is released.

When the centrifuge tube 12 is returned from the centrifugation section 30 to the rack transporter 20, a prepared specimen is stored in the centrifuge tube 12. Therefore, it is necessary to avoid the centrifuge tube 12 from falling out during transfer so that the specimen does not spill into the apparatus. Thus, the transfer unit 40 illustrated in FIG. 5 has a spring member 440 that biases the grippers 434, 435 in a direction of closing each other. As a result, the grippers 434, 435 can remain closed even without the driving force of the motor, so that even in the case that an abnormality occurs in the open/close motor 420, the centrifuge tube 12 can be prevented from falling off during transfer.

Furthermore, in the transfer unit 40 illustrated in FIG. 5, the open/close motor 420 is attached to the sheet metal 417 so that the Z axis movement is not required. The driving force of the open/close motor 420 is transmitted to the slide section 432 by rotation of the vertically extending rods 423, 424. This structure is advantageous in that an element to move in the Z axis can be made lighter. That is, in this structure of the transfer unit 40, elements to move in the Z axis for transferring the centrifuge tube 12 are only the sheet metal 431, slide section 432 and a pair of grippers 434, 435. Since a heavy part such as the open/close motor 420 does not need to be moved in the Z axis, the load on the Z axis motor 416 can be reduced, and the possibility of failure due to disconnection of the Z axis motor 416 can be reduced. Also, even if an abnormality occurs in the Z axis motor 416, the centrifuge tube 12 being transferred together with the grippers 434, 435 can be avoided from falling down due to the weight reduction.

The specimen preparation apparatus 100 is provided with the transfer unit 40 of such a configuration so that the centrifuge tube 12 set in the rack 10b of the rack transporter 20 can be transferred to the rotor 31 of the centrifugation section 30. Therefore, there is no need for a user to access the centrifugation section 30. Thus, automation of the operation is achieved in comparison with the conventional technology in which the user sets the centrifuge tube 12 in the centrifugation section 30. Furthermore, by restricting the user's access to the centrifugation section 30, an improvement in safety is achieved.

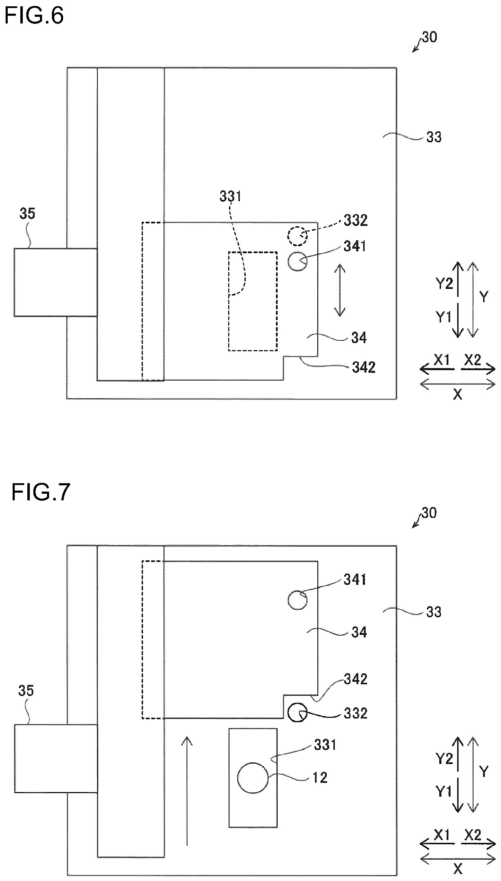

Referring to FIG. 6 to FIG. 8, a lid 34 that covers the upper part of the centrifugation section 30 is described. As illustrated in FIG. 6, the centrifugation section 30 is covered at the top by a cover 33. The cover 33 is provided with openings 331 and 332. The opening 331 is used for inserting and removing the centrifuge tube 12 into and out of the centrifugation section 30. Also, the opening 332 is used for dispensing a reagent into the centrifuge tube 12 in the centrifugation section 30 and for aspirating a specimen from the centrifuge tube 12. Since the opening 331 needs to pass through the centrifuge tube 12 and the transfer unit 40, the opening 331 has an area larger than the centrifuge tube 12. Also, the opening 332 has a smaller area than the centrifuge tube 12 because only the nozzle needs to pass through the opening 332.

Above the cover 33, a lid 34 is provided to close the openings 331 and 332. The lid 34 can slide in the horizontal direction (Y direction). The lid 34 is provided with an opening 341 and a notch 342. Also, the lid 34 is moved in the Y direction by a drive unit 35.

As illustrated in FIG. 6, the lid 34 moves in the Y1 direction to cover both the openings 331 and 332 of the cover 33. Specifically, the lid 34 covers the upper side of the opening 331. In addition, the opening 341 of the lid 34 is shifted in the Y1 direction with respect to the opening 332 of the cover 33, thereby covering the opening 332. The lid 34 normally keeps the openings 331 and 332 closed as illustrated in FIG. 6, except when transferring the centrifuge tube 12 by the transfer unit 40 or dispensing a sample/reagent by the dispenser 60.

Also, as illustrated in FIG. 7, the lid 34 moves in the Y2 direction to open both the openings 331 and 332 of the cover 33. Specifically, an end of the lid 34 on the Y1 direction side is moved to the Y2 direction side from the end of the opening 331 of the cover 33 on the Y2 direction side. In addition, the notch 342 in the lid 34 is moved to a position corresponding to the opening 332 in the cover 33, and the opening 332 is released. The lid 34 takes a position with the opening 331 opened illustrated in FIG. 7 when the transfer unit 40 sets the centrifuge tube 12 in the rotor 31 and when the transfer unit 40 removes the centrifuge tube 12 in the rotor 31. An operation when setting the centrifuge tube 12 is as follows. First, before the lid 34 opens, the rotor 31 rotates and stops so that the holder 32, in which the centrifuge tube 12 is set, is provided below the opening 331. When the transfer unit 40 gripping the centrifuge tube 12 is provided above the centrifugation section 30, the lid 34 moves to the state illustrated in FIG. 7 and opens the opening 331. Once the centrifuge tube 12 is set by the transfer unit 40, the lid 34 returns to the state illustrated in FIG. 6 and closes the opening 331. An operation of removing the centrifuge tube 12 is as follows. First, before the lid 34 opens, the rotor 31 rotates and stops so that the holder 32 holding the centrifuge tube 12 to be removed is provided below the opening 331. When the transfer unit 40 is provided above the centrifugation section 30, the lid 34 moves to the state illustrated in FIG. 7 and opens the opening 331. When the centrifuge tube 12 is removed by the transfer unit 40, the lid 34 returns to the state illustrated in FIG. 6 and closes the opening 331. Thus, since the lid 34 opens only when the transfer unit 40 accesses the centrifugation section 30, the user is prevented from accidentally putting his or her hand into the centrifugation section 30, thereby improving safety. In addition, the interior of the centrifugation section 30 is maintained at a constant temperature suitable for the reaction of the sample and the reagent. Therefore, by opening the lid 34 only when necessary, a fluctuation in the temperature inside the centrifugation section 30 is reduced. In addition, whenever the opening 331 is opened by the lid 34, the rotation of the rotor 31 is stopped, which further improves safety.

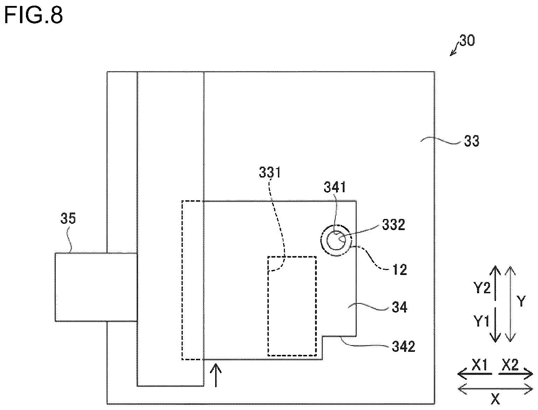

Also, as illustrated in FIG. 8, the lid 34 moves in the Y2 direction to a small extent to open the opening 332 of the cover 33 while closing the opening 331 of the cover 33. Specifically, the lid 34 covers the upper part of the opening 331. In addition, the opening 341 of the lid 34 is provided in a position corresponding to the opening 332 of the cover 33, and the opening 332 is released. The lid 34 takes a position with the opening 332 opened illustrated in FIG. 8 when the transfer unit 40 dispenses a sample or a reagent to the centrifuge tube 12 held by the holder 32. An operation when dispensing a sample or reagent is as follows. First, before the lid 34 opens, the rotor 31 rotates and stops so that the holder 32 holding the centrifuge tube 12 into which a sample or a reagent is dispensed is provided below the opening 331. When the dispenser 60 is provided above the centrifugation section 30, the lid 34 moves to the state illustrated in FIG. 8 and opens the opening 332. Once the sample or a reagent is dispensed into the centrifuge tube 12 by the dispenser 60, the lid 34 returns to the state illustrated in FIG. 6 and closes the opening 332. Although dispensing a sample or a reagent is described here, an operation when liquid (e.g., supernatant) is removed from the centrifuge tube 12 by aspiration is also the same.

### [Operation of the specimen preparation apparatus]

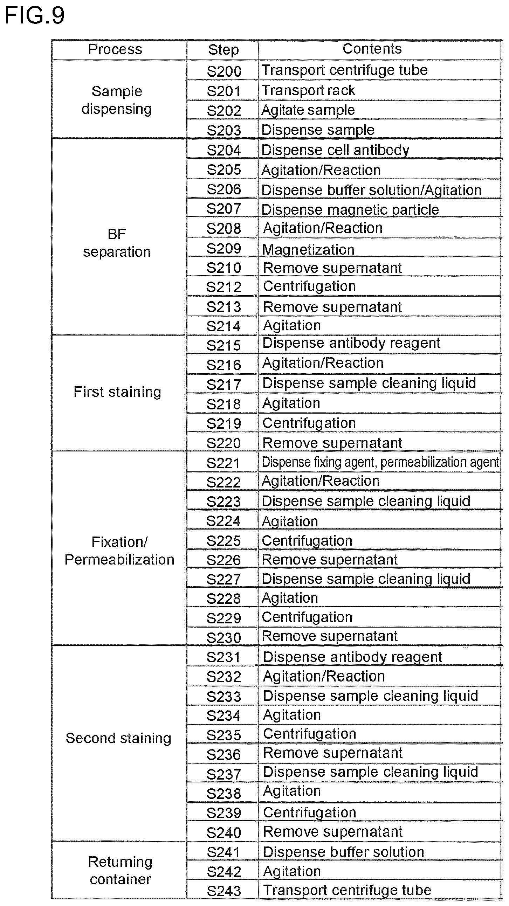

Referring to FIG. 9, an operation of a measurement specimen by the specimen preparation apparatus 100 is described. In the following, FIG. 10 to FIG. 13 are also referred to regarding a movement of the racks 10a to 10c by the rack transporter 20.

As illustrated in FIG. 10, the racks 10a, 10b, and 10c can each hold a plurality (e.g., six) of containers. The racks 10a, 10b, and 10c are transported in the X1 direction and the X2 direction by the rack transporter 20. This embodiment describes an embodiment, in which the rack transporter 20 moves the racks in the X1 direction and the X2 direction integrally, but the rack transporter 20 may move the racks independently of each other. The rack transporter 20 can transport the racks 10a, 10b, 10c in the X1 direction and the X2 direction by distances corresponding to the spacing of containers held in the racks. In FIG. 10 to FIG. 13, in order to clearly illustrate the position of each container, squares of a size corresponding to the interval between the containers are illustrated.

### <Process of dispensing a sample>

Prior to a process of dispensing a sample, as illustrated in FIG. 10A, containers are set by the user on the racks 10a to 10c in an initial position, respectively. The initial position of the racks 10a to 10c means that each rack is at a rightmost position (X2 direction side). The user sets an empty processing container 11 in the rack 10a. Also, the user sets an empty centrifuge tube (reaction container) 12 in the rack 10b. Also, the user sets a sample container 13 storing a blood sample in the rack 10c.

In step S201 of FIG. 9, the centrifuge tube 12 as a reaction container is transported from the rack 10b to the centrifugation section 30. As illustrated in FIG. 10B, the rack is moved in the X1 direction so that the leftmost centrifuge tube 12 is located at a position P1. The position P1 is the twelfth square from the rightmost position. At the position P1, the centrifuge tube 12 is taken out by the transfer unit 40 and transferred to the centrifugation section 30. As illustrated in FIG. 10C, the rack is moved in the X1 direction so that the next centrifuge tube 12 is located at the position P1. Then, the centrifuge tube 12 is taken out by the transfer unit 40 and sequentially transferred to the centrifugation section 30. This process is repeated until the last centrifuge tube 12 is moved to the position P1 and transferred to the centrifugation section 30 by the transfer unit 40. The controller 90 stores a correspondence between each holder 32 of the rotor 31 and a holding position in the rack 10b of the centrifuge tube 12 set in each holder 32.

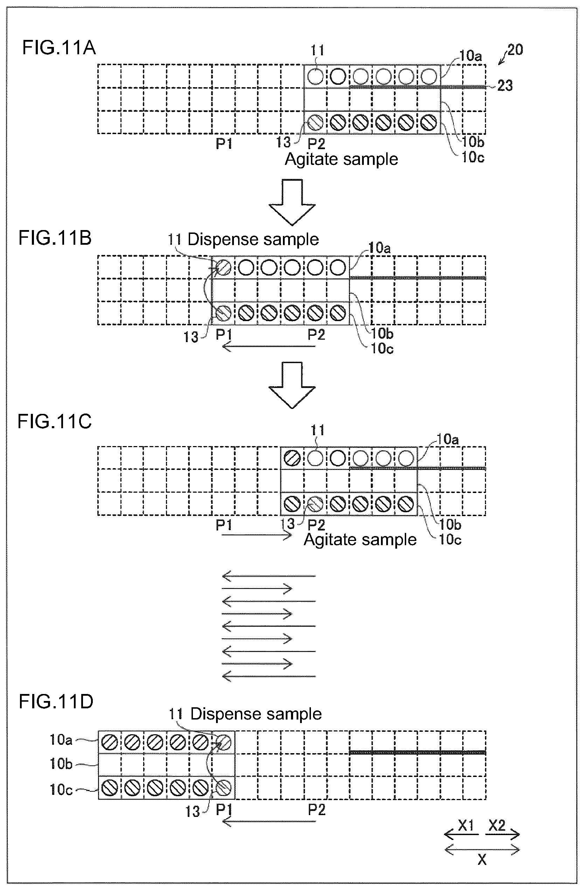

In step S202, a blood sample in the sample container 13 is agitated. As illustrated in FIG. 11A, the rack is moved in the X2 direction by the rack transporter 20 so that the leftmost sample container 13 is located at a position P2. The position P2 is the eighth square from the rightmost position. At the position P2, the sample container 13 of the rack 10c is removed by the agitator 51 and is inverted and agitated.

In step S203, a blood sample is aspirated from the sample container 13 and discharged into the processing container 11 of the rack 10a, and the blood sample is dispensed. As illustrated in FIG. 11B, the rack is moved in the X1 direction so that the sample container 13 that is agitated in step S202 is located at the position P1. At the position P1, the dispenser 50 aspirates a portion of the blood sample in the sample container 13 and dispenses the aspirated blood sample into the processing container 11 held in the rack 10a. As illustrated in FIG. 11C, the rack is moved in the X2 direction so that the next sample container 13 is located at the position P2. At the position P2, the sample container 13 is agitated by the agitator 51. This agitating of the sample container 13 at the position P2 and the subsequent dispensing of the blood sample at the position P1 are repeated until all sample containers 13 are dispensed. When a sample is dispensed from the sample containers 13, the sample in the sample container 13 held in one holding position in the rack 10c is dispensed into the corresponding into the processing container 11 held in the corresponding holding position of the rack 10a. The corresponding holding position means the nth holding position from the end (rightmost or leftmost) of one rack and the nth holding position from the same side of another rack. For example, a sample in the sample container 13 held in the leftmost holding position of the rack 10c is dispensed into the processing container 11 held in the corresponding holding position of the rack 10a, i.e., the leftmost holding position. Other samples are dispensed in the same manner. FIG. 11D is a diagram illustrating a state in which agitating and dispensing for all sample containers 13 are completed.

### <BF separation process>

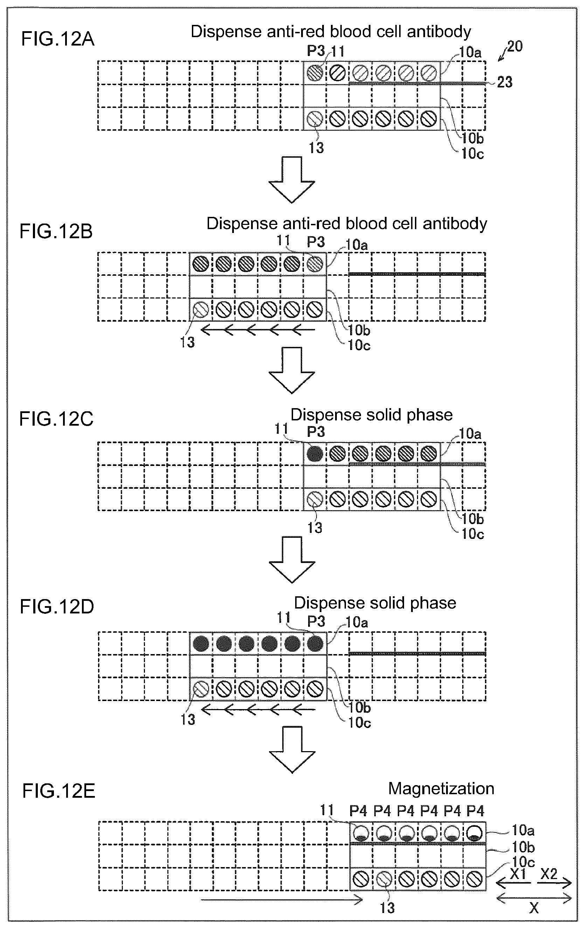

In step S204, an antibody is dispensed into the processing container 11 from which the blood sample is discharged. As illustrated in FIG. 12A, the rack is moved in the X2 direction so that the leftmost processing container 11 is located at a position P3. At the position P3, the dispenser 60 dispenses a biotinylated anti-red blood cell antibody into the processing container 11. As illustrated in FIG. 12B, the rack is moved in the X1 direction so that the next processing container 11 is located at the position P3. Then, at the position P3, the process of dispensing the anti-red blood cell antibody into the processing container 11 is repeated by the dispenser 60.

When dispensing is completed to all the processing containers 11, in step S205, an agitation of the blood sample in the processing container 11 is performed. The rack is repeatedly moved back and forth in the X1 direction and the X2 direction by the rack transporter 20, and the blood sample in the processing container 11 is agitated. The rack 10a holding the processing container 11 is then allowed to stand still for a predetermined period of time (e.g., 20 minutes).

In step S206, a buffer solution is dispensed into the processing container 11. The rack transporter 20 moves the rack 10a, and at the position P3, a buffer is dispensed into each processing container 11 by the dispenser 60. The rack 10a is moved at a high speed by the rack transporter 20, and the blood sample in the processing container 11 is agitated. Thereafter, the blood sample is allowed to stand still. For example, a BSA solution and a phosphate buffered saline (PBS) are dispensed as buffer solutions. Also, the blood sample to which the buffer solution is dispensed is agitated.

In step S207, a magnetic particle is dispensed into the processing container 11. For example, a streptavidin-bound magnetic particle is dispensed as a magnetic particle. As illustrated in FIG. 12C, the rack is moved in the X1 direction so that the leftmost processing container 11 is located at the position P3. At the position P3, the streptavidin-bound magnetic particle as a solid phase substance is dispensed into the processing container 11 by the dispenser 60. As illustrated in FIG. 12D, the rack is moved in the X1 direction so that the next processing container 11 is located at the position P3. Then, at the position P3, the process of dispensing the solid phase substance including a magnetic particle into the processing container 11 by the dispenser 60 is repeated.

In step S208, the blood sample in the processing container 11 is agitated, and a reaction is carried out. The time required is, for example, 5 minutes. The rack is moved at a high speed by the rack transporter 20, and the blood sample in the processing container 11 is agitated. Then, the blood sample is allowed to stand still. Therefore, a complex including red blood cells and a solid phase substance is formed in the processing container 11.

In step S209, magnetic collection is performed. As illustrated in FIG. 12E, the rack is moved in the X2 direction so that each processing container 11 is located at a position P4. The positions P4 are positions corresponding to six squares from the rightmost square. A magnet 23 is provided at a position adjacent to the position P4. The magnet 23 causes the complex including the red blood cells and the solid phase substance to be attracted on the inner surface of the processing container 11. The time required is, for example, 10 minutes.

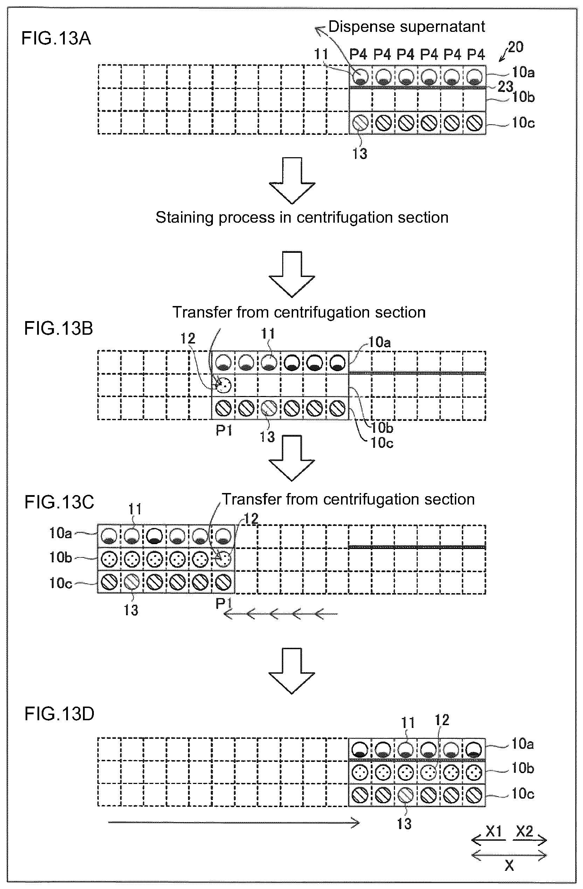

In step S210, a supernatant (e.g., 700 µL) of the blood sample in the processing container 11, in which magnetic attraction is performed, is aspirated and discharged into the centrifuge tube 12 of the centrifugation section 30 to be dispensed. As illustrated in FIG. 13A, at the position P4, the supernatant is aspirated from each processing container 11 by the dispenser 60 and dispensed into each centrifuge tube 12 transferred to the centrifugation section 30. When the supernatant (sample) is dispensed from the processing container 11, the supernatant (sample) of the processing container 11 held in one holding position of the rack 10a is dispensed into the centrifuge tube 12 held in the corresponding holding position of the rack 10b. The corresponding holding position is as described previously. For example, the supernatant (sample) of the processing container 11 held at the leftmost holding position of the rack 10a is dispensed into the processing container 11 held in the corresponding holding position of the rack 10a before transferring to the centrifugation section 30 (i.e., at the time of FIG. 10A), that is, to the leftmost holding position. As described above, the controller 90 stores a correspondence between each holder 32 of the rotor 31 and the holding position in the rack 10b of the centrifuge tube 12 set in each holder 32. The dispenser 60, based on the correspondence stored in the controller 90, dispenses the supernatant (sample) of the centrifuge tube 12 held in one holding position in the rack 10a to the centrifuge tube 12 held in the corresponding holder 32 (i.e., the centrifuge tube 12 held in the corresponding holding position in the rack 10b). Other samples are dispensed in the same manner.

In step S212, the specimen in the centrifuge tube 12 is centrifuged. In the centrifugation section 30, the rotor 31 is rotated at a high speed to settle the white blood cell at the bottom of the centrifuge tube 12.

In step S213, the supernatant in the centrifuge tube 12 is removed. The dispenser 60 aspirates and removes the supernatant (e.g., 600 µL) of the centrifuge tube 12, in which the white blood cell is settled by centrifugation.

In step S214, the specimen in the centrifuge tube 12 is agitated. The white blood cell is settled at the bottom of the centrifuge tube 12 by step S212 and step S213. In order to disperse the settled white blood cell, the centrifuge tube 12 is agitated. The centrifugation section 30 agitates the specimen in the centrifuge tube 12 by rotating the rotor 31 in one direction with repeated acceleration and deceleration. In this embodiment, repeated rotation with acceleration and deceleration in one direction as agitation by the centrifugation section 30 is explained as an example, but a rotation method for agitation may be intermittent rotation or rotation in both forward and reverse directions.

### <First staining process>

In step S215, an antibody reagent is dispensed into the centrifuge tube 12. The dispenser 60 dispenses a cocktail reagent including a CD25-labeled antibody, a CD4-labeled antibody, and a CD62-labeled antibody as the antibody reagent into the centrifuge tube 12 held in the centrifugation section 30.

In step S216, the centrifugation section 30 rotates the rotor 31 while repeatedly accelerating and decelerating in one direction to agitate the specimen in the centrifuge tube 12, and a reaction of the specimen proceeds. The predetermined time is, for example, 30 minutes.

In step S217, a cleaning liquid for the pre-fixation sample is dispensed into the centrifuge tube 12. The dispenser 60 dispenses a PBS as the cleaning liquid into the centrifuge tube 12.

In step S218, the centrifugation section 30 agitates the specimen in the centrifuge tube 12 by rotating the rotor 31 while repeatedly accelerating and decelerating in one direction.

In step S219, the centrifugation section 30 centrifuges the specimen in the centrifuge tube 12 by rotating the rotor 31 at a high speed in one direction. As a result, the white blood cell that reacts with the antibody reagent is settled.

In step S220, the dispenser 60 aspirates and removes the supernatant from the centrifuge tube 12. As a result, the surface antigens CD25, CD4 and CD62 are stained with the corresponding labeled substances.

### <Processes of fixation and permeabilization of cells>

In step S221, the dispenser 60 dispenses a fixation/permeabilization agent into the centrifuge tube 12.

In step S222, the centrifugation section 30 agitates the specimen in the centrifuge tube 12 by rotating the rotor 31 while repeatedly accelerating and decelerating in one direction, and the reaction is performed. The predetermined time is, for example, 30 minutes.

In step S223, the dispenser 60 dispenses a cleaning liquid for the post-fixation sample into the centrifuge tube 12.

In step S224, the centrifugation section 30 agitates the specimen in the centrifuge tube 12 by rotating the rotor 31 while repeatedly accelerating and decelerating in one direction.

In step S225, the centrifugation section 30 centrifuges the specimen in the centrifuge tube 12 by rotating the rotor 31 at a high speed.

In step S226, the dispenser 60 aspirates and removes the supernatant from the centrifuge tube 12.

In steps S227 to S230, dispensing of the cleaning liquid, agitation, centrifugation and removal of the supernatant are performed. In other words, a cleaning process of the sample is repeated. The cleaning process of the sample may be performed once, twice, three or more times. As a result, processes of fixation and permeabilization for the cell in the centrifuge tube 12 are performed.

### <Second staining process>

In step S231, the dispenser 60 dispenses an antibody reagent into the centrifuge tube 12. For example, a reagent including a Foxp3-labeled antibody is dispensed as the antibody reagent.

In step S232, the centrifugation section 30 rotates the rotor 31 while repeatedly accelerating and decelerating in one direction to agitate the specimen in the centrifuge tube 12 and perform a reaction. The predetermined time is, for example, 30 minutes.

In step S233, the dispenser 60 dispenses a cleaning liquid for the post-fixation sample into the centrifuge tube 12. In steps S234 to S236, as described above, agitation, centrifugation and removal of the supernatant are performed, and in steps S237 to S240, a process of cleaning the sample including dispensing of the cleaning liquid, agitation, centrifugation, and removal of the supernatant is repeated. The process of cleaning the sample may be performed once, twice, three or more times. As a result, the FOXP3 in the centrifuge tube 12 is stained with the corresponding labeled substance.

### <Returning the container>

In step S241, the dispenser 60 dispenses a buffer solution into the centrifuge tube 12. Dispensing adjusts the specimen in the centrifuge tube 12 to a predetermined volume of liquid and a predetermined ph suitable for supply to the measurement apparatus. For example, a BSA solution and a PBS are dispensed as buffer solutions.

In step S242, the centrifugation section 30 rotates the rotor 31 to perform agitation as described above.

In step S243, the transfer unit 40 removes the centrifuge tube 12 held in the centrifugation section 30 from the rotor 31 and sets the centrifuge tube 12 on the rack 10b held in the rack transporter 20. At this time, each centrifuge tube 12 is returned to its original position in the rack 10b. As described above, the controller 90 stores a correspondence between each holder 32 of the rotor 31 and the holding position in the rack 10b of the centrifuge tube 12 set in each holder 32. The transfer unit 40 transfers the centrifuge tube 12 held in each holder 32 back to its original holding position in the rack 10b based on the correspondence stored in the controller 90. For example, the centrifuge tube 12 that is held in the leftmost holding position in the initial position in FIG. 10A is set in its original holding position (leftmost holding position) when the centrifuge tube 12 is returned to the rack 10b as illustrated in FIG. 13B. The other centrifuge tubes 12 are similarly returned to their original holding positions in the rack 10b. When all the centrifuge tubes 12 are transferred to the rack 10b, as illustrated in FIG. 13D, the rack is moved in the X2 direction by the rack transporter 20 to return to the initial position. This enables the user to remove the centrifuge tube 12. As described above, the user does not need to access the centrifugation section 30 not only when setting the centrifuge tube 12 in the centrifugation section 30 but also when taking out the centrifuge tube 12; therefore, a high level of safety is ensured. Furthermore, the centrifuge tube 12 storing the prepared specimen is returned to the original holding position of the rack 10b, so that the user can remove the centrifuge tube 12 while maintaining the correspondence between the holding position of the sample container 13 in the initial position and the holding position of the centrifuge tube 12. Therefore, it is easy for the user to understand which sample container 13 corresponds to which centrifuge tube 12, and the risk of mistakenly removing a specimen can be reduced.

The above completes the specimen preparation using the specimen preparation apparatus 100.

### (Operation process of the centrifugation section of the specimen preparation apparatus)

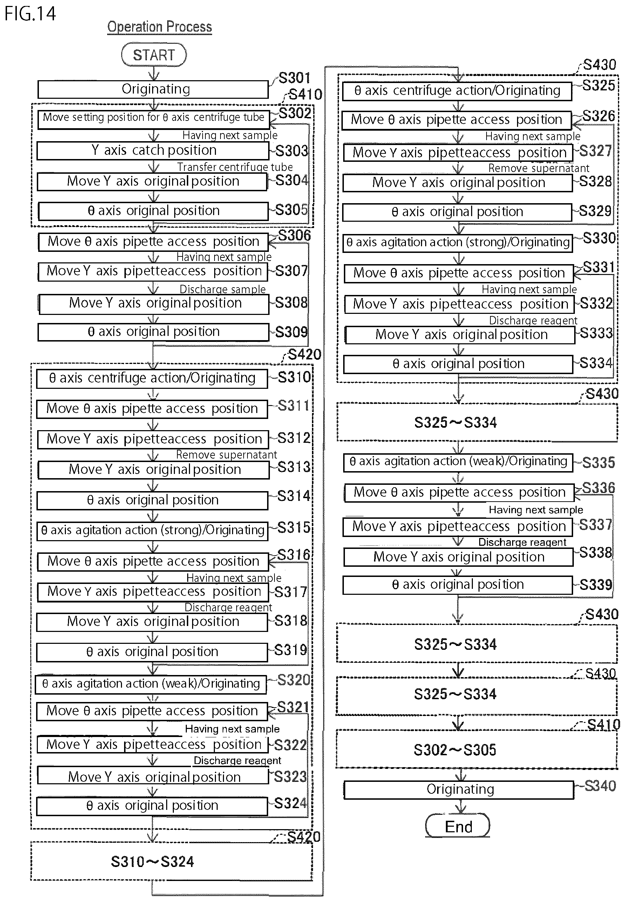

Next, with reference to FIG. 14, an operation process of the centrifugation section 30 of the specimen preparation apparatus 100 is described.

The centrifugation section 30 positions the rotor 31 at a desired rotational position by adjusting a position of the rotor 31 at the origin and the rotational distance from the origin.

In step S301, the origin of the rotor 31 of the centrifugation section 30 is set out. Thereafter, a process of step S410 (steps S302 to 305) is processed.

As step S410, first, in step S302, a θ axis of the rotor 31 is moved to a set position of the centrifuge tube 12 as a reaction container. Specifically, a rotation angle of the rotor 31 is aligned with a position to which the centrifuge tube 12 is transferred. In step S303, a Y axis of the transfer unit 40 is moved to a catch position. Then, the centrifuge tube 12 is caught by the transfer section 40, and the centrifuge tube 12 is transferred to the rotor 31.

Thereafter, in step S304, the Y axis of the transfer unit 40 is moved to an origin position. In step S305, the θ axis of the rotor 31 is moved to the origin position. When there is a next sample, process of steps S302 to S305 (step S410) are repeated.

In step S306, the θ axis of the rotor 31 is moved to a pipette access position where the sample is dispensed, the reagent is dispensed or the supernatant is aspirated. In step S307, an XY axis of the dispenser 60 is moved to the pipette access position. Then, the sample is discharged into the centrifuge tube 12 of the rotor 31 by the dispenser 60.

Thereafter, in step S308, the XY axis of the dispenser 60 is moved to the origin position. In step S309, the θ axis of the rotor 31 is moved to the origin position. When there is a next sample, processes of steps S306 to S309 are repeated.

Thereafter, the process of step S420 (steps S310 to S324) is repeated twice.

As step S420, first, in step S310, a process of centrifugation is performed, followed by originating of the rotor 31. In step S311, the θ axis of the rotor 31 is moved to the pipette access position. In step S312, the XY axis of the dispenser 60 is moved to the pipette access position. Then, the supernatant is aspirated and removed from the centrifuge tube 12 of the rotor 31 by the dispenser 60.

In step S313, the XY axis of the dispenser 60 is moved to the origin position. In step S314, the θ axis of the rotor 31 is moved to the origin position. In step S315, the θ axis of the rotor 31 is driven to perform a strong agitation operation. Thereafter, the originating of the rotor 31 is performed.

In step S316, the θ axis of the rotor 31 is moved to the pipette access position. In step S317, the XY axis of the dispenser 60 is moved to the pipette access position. Then, the reagent is dispensed into the centrifuge tube 12 of the rotor 31 by the dispenser 60.

In step S318, the XY axis of the dispenser 60 is moved to the origin position. In step S319, the θ axis of the rotor 31 is moved to the origin position. When there is a next sample, processes of steps S316 to S319 are repeated.

In step S320, the θ axis of the rotor 31 is driven to perform a weak agitation operation. Thereafter, the originating of the rotor 31 is performed.

In step S321, the θ axis of the rotor 31 is moved to the pipette access position. In step S322, the XY axis of the dispenser 60 is moved to the pipette access position. Then, the reagent is discharged into the centrifuge tube 12 of the rotor 31 by the dispenser 60.

In step S323, the XY axis of the dispenser 60 is moved to the origin position. In step S324, the θ axis of the rotor 31 is moved to the origin position. When there is a next sample, processes of steps S321 to S324 are repeated.

After the process of step S420 (steps S310 to S324) is repeated twice, a process of step S430 (steps S325 to S334) is repeated twice.

As step S430, first, in step S325, the process of centrifugation is performed, followed by originating of the rotor 31. In step S326, the θ axis of the rotor 31 is moved to the pipette access position. In step S327, the XY axis of the dispenser 60 is moved to the pipette access position. Then, the supernatant is aspirated and removed from the centrifuge tube 12 of the rotor 31 by the dispenser 60.

In step S328, the XY axis of the dispenser 60 is moved to the origin position. In step S329, the θ axis of the rotor 31 is moved to the origin position. When there is a next sample, processes of steps S326 to S329 are repeated.

In step S330, the θ axis of the rotor 31 is driven to perform a strong agitation operation. Thereafter, the originating of the rotor 31 is performed.

In step S331, the θ axis of the rotor 31 is moved to the pipette access position. In step S332, the XY axis of the dispenser 60 is moved to the pipette access position. Then, the reagent is discharged into the centrifuge tube 12 of the rotor 31 by the dispenser 60.

In step S333, the XY axis of the dispenser 60 is moved to the origin position. In step S334, the θ axis of the rotor 31 is moved to the origin position. When there is a next sample, processes of steps S331 to S334 are repeated.

After the process of step S430 (steps S325 to S334) is repeated twice, in step S335, the θ axis of the rotor 31 is driven to perform a weak agitation operation. Thereafter, the originating of the rotor 31 is performed.

In step S336, the θ axis of the rotor 31 is moved to the pipette access position. In step S337, the XY axis of the dispenser 60 is moved to the pipette access position. Then, the reagent is discharged into the centrifuge tube 12 of the rotor 31 by the dispenser 60.

In step S338, the XY axis of the dispenser 60 is moved to the origin position. In step S339, the θ axis of the rotor 31 is moved to the origin position. When there is a next sample, processes of steps S336 to S339 are repeated.

Thereafter, the process of step S430 (steps S325 to S334) is performed twice. Thereafter, step S410 (steps S302 to S305) is performed. In step S340, the originating of the rotor 31 of the centrifugation section 30 is performed, and the operation is terminated.

### (Another configuration example)

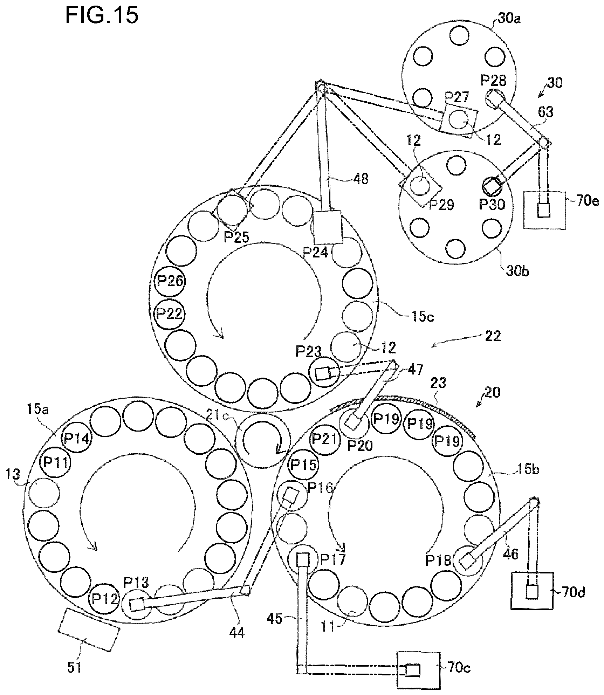

Referring to FIG. 15 to FIG. 17, an example of the operation of the preparation process of a measurement specimen by the specimen preparation apparatus 100 according to another configuration example is described.

As illustrated in FIG. 15, the specimen preparation apparatus 100 according to another configuration example includes a rack transporter 20 and a centrifugation section 30.

Also, the specimen preparation apparatus 100 includes a sample table 15a that rotates and transfers the sample container 13, a removal processing table 15b that rotates and transfers the processing container 11, and a container transporting table 15c that rotates and transfers the centrifuge tube 12 as the container transport mechanism 22.

The specimen preparation apparatus 100 includes a common drive unit 21c that rotates and moves the sample table 15a, the removal processing table 15b, and the container transporting table 15c around a rotation axis extending in the vertical direction, respectively.

Also, the specimen preparation apparatus 100 includes a dispenser 44, a dispenser 45, a dispenser 46, and a dispenser 47. The dispenser 44 is rotatable around the rotation axis extending in the vertical direction. In addition, the dispenser 44 is movable in the Z direction (vertical direction). Moreover, the dispenser 44 aspirates a blood sample from the sample container 13 of the sample table 15a and discharges the blood sample to the processing container 11 of the removal processing table 15b to dispense the blood sample.

The dispenser 45 is rotatable around the rotation axis extending in the vertical direction. Also, the dispenser 45 is movable in the Z direction (vertical direction). Moreover, the dispenser 45 aspirates the reagent installed in a reagent setting part 70c and discharges the reagent into the processing container 11 of the removal processing table 15b to dispense the reagent. The dispenser 46 is rotatable around the rotation axis extending in the vertical direction. Also, the dispenser 46 is movable in the Z direction (vertical direction). Moreover, the dispenser 46 aspirates the reagent installed in the reagent setting part 70d and discharges the reagent into the processing container 11 of the removal processing table 15b to dispense the reagent.

The dispenser 47 is rotatable around the rotation axis extending in the vertical direction. Also, the dispenser 47 is movable in the Z direction (vertical direction). Moreover, the dispenser 47 aspirates the supernatant after processing from the processing container 11 of the removal processing table 15b and discharges the supernatant to the centrifuge tube 12 of the container transporting table 15c to dispense the supernatant after processing of the blood sample.

The specimen preparation apparatus 100 also includes a container transfer unit 48. The container transfer unit 48 is rotatable around the rotation axis extending in the vertical direction. Also, the container transfer unit 48 is movable in the Z direction (vertical direction). The container transfer unit 48 transfers the centrifuge tube 12 between the container transporting table 15c and the centrifugation section 30.

In addition, the specimen preparation apparatus 100 includes an agitator 51 that agitates a blood sample. The agitator 51 holds and vibrates the sample container 13 to agitate the blood sample in the sample container 13.

Also, the specimen preparation apparatus 100 includes a dispenser 63. The dispenser 63 is rotatable around the rotation axis extending in the vertical direction. In addition, the dispenser 63 is movable in the Z direction (vertical direction). Moreover, the dispenser 63 aspirates the reagent installed in a reagent setting part 70e, discharges the reagent into the centrifuge tube 12 of the centrifugation section 30 to dispense the reagent. Also, the dispenser 63 aspirates and removes the supernatant from the blood sample in the centrifuge tube 12 of the centrifugation section 30.

Also, the specimen preparation apparatus 100 includes reagent setting parts 70c, 70d and 70e.

The rack transporter 20 forms a complex including red blood cells and a solid phase substance and includes a magnet 23 that separates the formed complex from the supernatant of the blood sample. The magnet 23 is, for example, a BF separation section that performs BF separation.

The centrifugation section 30 is, for example, a centrifugation section capable of performing centrifugation. The centrifugation section 30 includes a first centrifugation section 30a and a second centrifugation section 30b. The first centrifugation section 30a and the second centrifugation section 30b are driven independently of each other and process the blood sample with a batch processing.

In the sample table 15a, the sample container 13 is set at a position P11. The specimen container 13 set at the position P11 is rotated counterclockwise as shown in FIG. 15 and moved to a position P12. The sample container 13 at the position P12 is agitated by the agitator 51. The sample container 13 agitated by the agitator 51 is moved to a position P13, and the blood sample is aspirated by the dispenser 44. Thereafter, the sample container 13 is sequentially moved to a position P14 as the next sample container 13 is processed. The sample container 13 that is moved to the position P14 is removed. Setting the sample container 13 at the position P11 and removing the sample container 13 from the position P14 may be performed by the user or by a separately provided sample transporting apparatus.

In the removal processing table 15b, the processing container 11 is set at a position P15. The processing container 11 set at the position P15 is rotated counterclockwise as shown in FIG. 15 and moved to a position P16. The blood sample is dispensed into the processing container 11 at the position P16 by the dispenser 44. The processing container 11 to which the blood sample is discharged is moved to a position P17, and a reagent is discharged by the dispenser 45. Thereafter, the processing container 11 is moved to a position P18, and a reagent is discharged by the dispenser 46. Thereafter, the processing container 11 is moved to a position P19, and the BF separation process is performed by the magnet 23 of the rack transporter 20. Then, the processing container 11 is moved to a position P20, and the supernatant is aspirated by the dispenser 47. Thereafter, the processing container 11 is moved to a position P21 and removed. Setting the processing container 11 at the position P15 and removing the processing container 11 from the position P21 may be performed by the user or by a separately provided container transporting apparatus.

In the container transporting table 15c, the centrifuge tube 12 is set at a position P22. The centrifuge tube 12 set at the position P22 is rotated counterclockwise as shown in FIG. 15 and moved to a position P23. In the centrifuge tube 12 at the position P23, the supernatant of the blood sample is discharged by the dispenser 47. The centrifuge tube 12 into which the supernatant of the blood sample is discharged is moved to a position P24. The centrifuge tube 12 at the position P24 is transferred to the centrifugation section 30 by the container transfer unit 48. For example, the container transfer unit 48 transfers the centrifuge tube 12 to the first centrifugation section 30a when there is a vacancy in the first centrifugation section 30a. On the other hand, when there is no space in the first centrifugation section 30a, the container transfer unit 48 transfers the centrifuge tube 12 to the second centrifugation section 30b. The centrifuge tube 12 processed by the centrifugation section 30 is transferred to a position P25 of the container transporting table 15c by the container transfer unit 48. Thereafter, the centrifuge tube 12 is moved to a position P26 and is removed. Setting the centrifuge tube 12 at the position P22 and removing the centrifuge tube 12 from the position P26 may be performed by the user or by a separately provided container transporting apparatus.

In the first centrifugation section 30a of the centrifugation section 30, the centrifuge tube 12 is transferred to a position P27 by the container transfer unit 48. Also, at a position P28, a reagent is discharged into the centrifuge tube 12 by the dispenser 63. Also, at the position P28, the supernatant is aspirated from the centrifuge tube 12 by the dispenser 63.

In the second centrifugation section 30b of the centrifugation section 30, the centrifuge tube 12 is transferred to a position P29 by the container transfer unit 48. Also, at a position P30, a reagent is discharged into the centrifuge tube 12 by the dispenser 63. Also, at the position P30, the supernatant is aspirated from the centrifuge tube 12 by the dispenser 63.

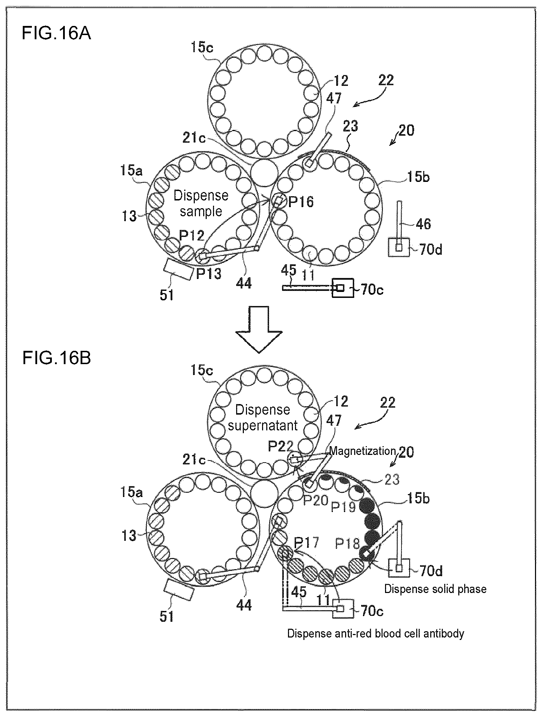

As illustrated in FIG. 16A, the sample container 13 of the sample table 15a is moved to the position P12. At the position P12, the sample container 13 is agitated by the agitator 51. Thereafter, the sample container 13 agitated by the agitator 51 is moved to the position P13, and the blood sample is aspirated by the dispenser 44. The blood sample aspirated at the position P13 is discharged into the processing container 11 of the removal processing table 15b at the position P16 by the dispenser 44 to be dispensed.

As illustrated in FIG. 16B, the processing container 11 of the removal processing table 15b, to which the blood sample is dispensed, is moved to the position P17, and the anti-red blood cell antibody is dispensed by the dispenser 45. Thereafter, the processing container 11 is moved to the position P18, and the solid phase substance including a buffer and a magnetic material is dispensed by the dispenser 46. Thereafter, the processing container 11 is moved to the position P19, and magnetic attraction is performed by the magnet 23 of the rack transporter 20. Then, the processing container 11 is moved to the position P20, and the supernatant is aspirated by the dispenser 47. The supernatant aspirated at the position P20 is discharged and dispensed into the centrifuge tube 12 of the container transporting table 15c at the position P22 by the dispensing section 47.

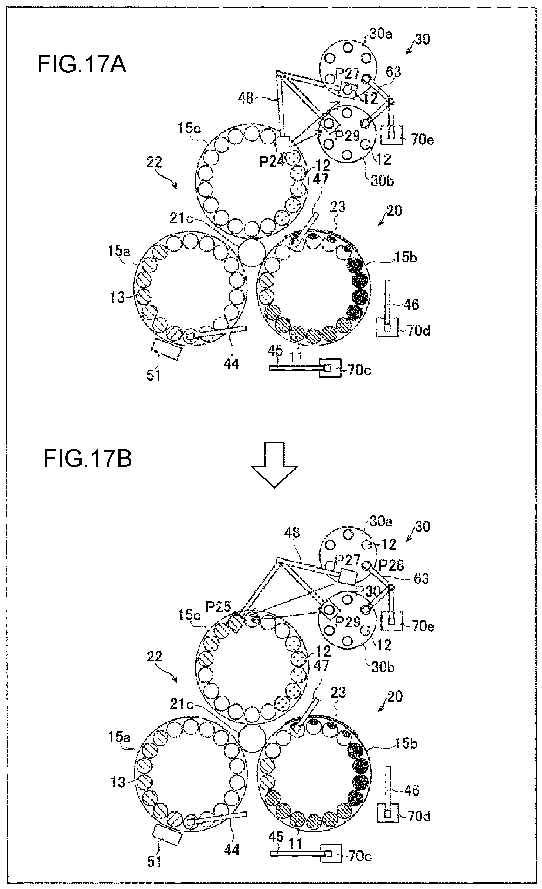

As illustrated in FIG. 17A, the centrifuge tube 12, into which the supernatant of the blood sample is discharged, is moved to the position P24. The centrifuge tube 12 at the position P24 is transferred to the centrifugation section 30 by the container transfer unit 48. The centrifuge tube 12 at the position P24 is transferred to the position P27 of the first centrifugation section 30a or the position P29 of the second centrifugation section 30b.

In the centrifugation section 30, the process of dispensing the reagent, the process of centrifugation, and the process of removing the supernatant are repeated to immunostain cells of the blood sample.

As illustrated in FIG. 17B, in the first centrifugation section 30a of the centrifugation section 30, the immunostained centrifuge tube 12 is moved to the position P27. The centrifuge tube 12 at the position P27 is transferred to the container transporting table 15c by the container transfer unit 48. Also, in the second centrifugation section 30b of the centrifugation section 30, the immunostained centrifuge tube 12 is moved to the position P29. The centrifuge tube 12 at the position P29 is transferred to the container transporting table 15c by the container transfer unit 48.

### (Variation)

The presently disclosed embodiments should be considered exemplary and not restrictive in all respects. The scope of the invention is indicated by the claims rather than by the description of the above-described embodiments, and further includes all modifications within the meaning and scope equivalent to the claims.

For example, the above embodiment illustrates the rack transporter 20 that transports the racks 10a to 10c along the X axis as a holder that holds the centrifuge tube 12 to be set by the user, but the form of the holder is not limited thereto. For example, the holder and the centrifuge tube 12 held in the holder may not move within the apparatus. For example, the transfer unit 40 may be configured to move to the holding position where the user sets the centrifuge tube 12, take out the centrifuge tube 12, and transfer the centrifuge tube 12 to the centrifugation section 30.

In the embodiment described above, the centrifuge tube 12 after centrifugation is returned to the rack transporter 20, but the centrifuge tube 12 may be transferred to another location within the apparatus. For example, a specimen storage other than the rack transporter 20 may be provided in the apparatus, and the centrifuge tube 12 after centrifugation may be transferred there. In other words, the place where the centrifuge tube 12 is set by the user (i.e., the holder) and the place where the centrifuge tube 12 is taken out by the user after centrifugation may be separated.

In one or more embodiments described above, the dispenser 47 dispenses a sample into the centrifuge tube 12 transferred to the centrifugation section 30, but the dispenser 47 dispenses a sample into the centrifuge tube 12 held in the holder 32 before transferred by the transfer unit 40.

### [Description of the sign]

10a: Rack, 10b: Rack, 10c: Rack, 11: Processing container, 12: Centrifuge tube, 13: Sample container, 20: Holder, 21a, 21c: Drive unit, 22: Container transport mechanism, 23: Magnet, 30: Centrifugation section, 40: Transfer unit, 50: Dispenser, 51: Agitator, 60: Dispenser, 70a, 70b: Reagent setting part, 90: Controller, 100: Specimen preparation apparatus

## Claims

1. A specimen preparation apparatus to prepare a measurement specimen from a sample comprising:
a holder that holds a centrifuge tube;
a transfer unit that transfers the centrifuge tube held by the holder;
a dispenser that dispenses a sample into the centrifuge tube before or after being transferred by the transfer unit; and
a centrifugation section that holds the centrifuge tube transferred by the transfer unit and centrifuges a sample dispensed into the centrifuge tube by rotating the centrifuge tube.

2. The specimen preparation apparatus according to claim 1, wherein
the holder is arranged at a front side of the specimen preparation apparatus, and
the centrifugation section is arranged at a predetermined distance from the holder.

3. The specimen preparation apparatus according to claim 2, wherein
the centrifugation section is arranged at a rear side of the specimen preparation apparatus.

4. The specimen preparation apparatus according to any one of claims 1 to 3, wherein
the centrifugation section comprising:
an opening that allows access by the transfer unit; and
a lid that is movable to close the opening, wherein
the lid opens the opening in case that the transfer unit accesses the centrifugation section and closes the opening after transferring the centrifuge tube to the centrifugation section or removing the centrifuge tube from the centrifugation section by the transfer unit.

5. The specimen preparation apparatus according to claim 4, wherein
the centrifugation section stops rotating while the lid is opened.

6. The specimen preparation apparatus according to any one of claims 1 to 5, wherein
the dispenser dispenses a sample into the centrifuge tube transferred to the centrifugation section.

7. The specimen preparation apparatus according to any one of claims 1 to 5, wherein
the dispenser dispenses a sample into the centrifuge tube held in the holder before transferred by the transfer unit.

8. The specimen preparation apparatus according to any one of claims 1 to 7, wherein
the holder further holds a processing container different from the centrifuge tube, and
the dispenser comprises:
a first dispenser that dispenses a sample into the processing container;
and
a second dispenser that dispenses a sample processed in the processing container into the centrifuge tube.

9. The specimen preparation apparatus according to any one of claims 1 to 8, wherein the holder comprises:
a first holder that holds a plurality of sample containers; and
a second holder that holds a plurality of the centrifuge tubes.

10. The specimen preparation apparatus according to claim 9 further comprising:
a drive unit that moves the first holder to position the sample container in an aspirating position for aspiration by the dispenser.

11. The specimen preparation apparatus according to claim 10, wherein
the drive unit moves the second holder to position the centrifuge tube in a gripping position for the transfer unit to grip the centrifuge tube.

12. The specimen preparation apparatus according to any one of claims 1 to 11, wherein
the transfer unit returns the centrifuge tube to the holder after centrifugation.

13. The specimen preparation apparatus according to any one of claims 1 to 12, wherein
the transfer unit returns the centrifuge tube after centrifugation to an original holding position of the holder.

14. The specimen preparation apparatus according to claim 12 or 13, wherein
the holder comprises:
a first holder that holds a plurality of sample containers; and
a second holder that holds a plurality of the centrifuge tubes, and
the transfer unit returns the centrifuge tube that stores a specimen prepared from a sample in the sample container held at a first position of the first holder to a corresponding first position of the second holder, and the centrifuge tube that stores a specimen prepared from the sample container held in a second position of the first holder to a corresponding second position of the second holder.

15. The specimen preparation apparatus according to any one of claims 1 to 14, wherein
the holder holds the centrifuge tube stored in a case, and
the transfer unit transfers the centrifuge tube stored in the case by gripping the case and sets the centrifuge tube stored in the case in the centrifugation section.

16. The specimen preparation apparatus according to claim 15, wherein
the case comprises a thermally conductive metal.

17. The specimen preparation apparatus according to claim 15 or 16, wherein
the case has cylindrical shape and comprises a flange provided at an upper part of the cylindrical shape of the case, and
the transfer unit grips the case by engaging the flange.

18. The specimen preparation apparatus according to any one of claims 1 to 17, wherein
the transfer unit comprising:
a pair of grippers that grips the centrifuge tube;
a support member that supports the gripper;
a first drive source that horizontally moves the support member between at least a position above the holder and a position above the centrifugation section;
a second drive source that is fixed to the support member and vertically moves the gripper with respect to the support member; and
a third drive source that is fixed to the support member and opens and closes the gripper with respect to the support member.

19. A specimen processing apparatus comprising:
a container transport mechanism comprising:
a holder that holds a plurality of types of containers capable of storing a specimen; and
a drive unit that moves the holder;
a processing section that performs a process on the container or a specimen in the container; and
a controller, wherein
the controller controls the container transport mechanism and the processing section to move the plurality of types of containers in the same direction by the drive unit and to perform a predetermined process on at least one type of a container or a specimen in the container at a predetermined processing position for the at least one type of the container.

20. The specimen processing apparatus according to claim 19, wherein
the container transport mechanism comprises a common drive unit to move the plurality of types of containers, and
the common drive unit moves the plurality of types of containers the same distance simultaneously.

21. The specimen processing apparatus according to claim 19 or 20, wherein
the controller controls the container transport mechanism and the processing section to perform a different operation on a different type of container or a specimen in the container at the predetermined processing position.

22. The specimen processing apparatus according to any one of claims 19 to 21, wherein
the plurality of types of containers comprises at least one of a sample container to store a sample, a processing container to separate a specific component in a sample, and a reaction container to performing a reaction to stain cells in a sample.

23. A method of preparing a specimen comprising:
transferring a centrifuge tube to a centrifugation section;
dispensing a sample into the centrifuge tube transferred to the centrifugation section; and
centrifuging a sample stored in the centrifuge tube by the centrifugation section.

24. A method of processing a specimen using a specimen processing apparatus that comprises a container transport mechanism, which includes a holder to hold a plurality of types of containers capable of holding a specimen and a drive unit to move the holder, a processing section, which performs a process on the container or a specimen in the container, and a controller, comprising:
moving the plurality of types of containers in the same direction by the drive unit; and
performing a predetermined process on at least one type of a container or a specimen in the container at a predetermined processing position for the at least one type of container.
